# EUROPEAN PATENT APPLICATION

(11) **EP 1 426 028 A1**
(43) Date of publication of application: **09.06.2004**
(21) Application number: 02396180.8
(22) Date of filing: 03.12.2002
(51) Int. Cl.: A61K 7/00, A61K 35/10

(54) **Method of extract composition control in peat extraction, peat extract, and use of peat extract**

(71) Applicant: Aromtech Ltd, 95410 Tornio (FI)
(72) Inventor: Kallio, Heikki, Fin-21110 Naantali (FI); Korteniemi, Veli-Markku, Fin-94400 Keminmaa (FI); Tuomasjukka, Saska, Fin-21555 Taatila (FI); Määttä, Petri, Fin-95440 Kyläjoki (FI); Yang, Baoru,, Fin-20540Turku (FI); Judin, Vesa-Pekka, Fin-03100 Nummela (FI)
(74) Representative: Heikkilä, Hannes Antero

(57) **Abstract**

The present invention relates to a method of extract composition control in peat extraction by carbon dioxide, in which method the extraction temperature is 5 - 120 °C and pressure 70 - 1000 bar and the extraction is conducted in presence of a modifier. The invention relates also to a peat extract comprising 1 - 60 w-% sterols and use of it in skin care or keratin fibre care products, general hygiene products and/or cosmetic products.

## Description

The invention relates to a method of extract composition control in peat extraction and to peat extract defined in the preambles of the independent claims presented hereafter. Furthermore, the invention relates to use of a peat extract in skincare and keratin fibre care products, general hygiene products and cosmetic products.

Peat is a complex combination of partially degraded plant-derived biomass, varying in composition due to differences in peat-forming plants and stage of decomposition. Peat contains a variety of compounds soluble in organic solvents, known as the lipid or bitumen fraction, consisting of compounds usually categorised as waxes and resins, and further sterols, fatty acids, hydroxy acids, alkanes, alcohols, phenolic acids and terpenoids. Also humic substances, such as polyphenolic compounds, and aromatic compounds probably originating from lignine, fulvic substances and carbohydrates are found in peat. The exact composition of peat and its variation as well as the rationale for differences in peat compositions are basically unknown.

Peat has traditionally been used as such in peat baths and peat masks, which have been used to soothe, nourish and invigorate skin. Difficulties have been encountered in handling of dark coloured, tarnishing peat for skin care.

Both solvent extraction and super-critical fluid extraction methods for peat extraction have been used or proposed. Many methods use destructive steps, e.g. acid hydrolysis or extraction with hot liquids, which lead to both polymerisation and decomposition of organic compounds. Furthermore, most of the methods use organic solvents resulting in solvent residues in the final extracts.

In RU 2050853 is described a extraction of peat using carbon dioxide. The used extraction temperature was 25 - 29 °C, pressure 6.0 - 7.0 MPa and extraction time 2 - 3 hours.

An extraction of peat with an aliphatic solvent with at least 5 carbon atoms under conditions of elevated pressure and temperature for isolation of asphaltic materials was described in GB 2 001 670. There the preferred solvent was n-pentane with preferred temperatures starting from 40 % of the critical temperature of the solvent, which is for pentane about 80 °C.

Peat extracts have been proposed as ingredients in cosmetic formulations and medicinal products. Majority of peat extracts used have been defined as product-by-use or product-by-process, as it has not been possible to define the compounds in the extract. Peat extracts has been used in skin care based on the tradition without thorough knowledge of the bioactive components and effecting mechanism of peat. It is known that peat contains modest levels of lipophilic substances, of which plant sterols, long-chain alcohols and long-chain fatty acid esters are of particular interest with regard to their physiological effects especially on skin.

In WO 9216216 a peat-derived combination of water-soluble salts of cosmetically and pharmaceutically active compounds is proposed. DE 3402223 proposes a slightly coloured lipoid extract suitable for use in cosmetic products due to its visual properties, which extract was obtained from peat by extraction with myristinic acid isopropylester and/or by paraffin oil. US 4,272,527 proposes an ethanol extract of peat wax resin containing sterols and estrogenic compounds to be effective in treating of skin diseases. In WO 9858655 an extract containing humic acid is proposed to be used as a prophylactic to haemopoietic system injuries, the extract being produced with aqueous extraction of a specific peat type. In JP 63-222105 (A) an "extracted essence" isolated from low- to high-grade peat with supercritical CO₂ has been used in skin cosmetic compositions.

Sterols and steryl esters are important constituents of cell membranes and epidermal and sebaceous lipids. Incorporation of certain plant sterols into human keratinocyte plasma membrane have resulted in modulation of membrane fluidity as described by Mora et al., 1999. Topically applied phytosterols have been shown to be effective in improving dry skin by regulating and normalizing the turnover speed of corneocytes in the stratum corneum as described by Kashibuchi et al., 1993. Plant sterols have also shown anti-inflammatory and skin conditioning properties without significant hormone-like effects. This makes these compounds possible alternatives for partially replacing cortisone and corticosteroids in skin treatment. Phytosterols may also play a role in regulating immune functions of skin.

Long-chain alcohols and long-chain fatty acids are building materials of ceramides, a group of compounds essential for the lipid barrier structure of the stratum corneum of epidermis. Wax esters are important components of the lipid barrier structure. Aliphatic long-chain alcohols have been reported to be effective in promoting tissue regeneration and wound healing by Mironov et al, 1989.

It has not been possible to control the extract composition obtained from peat by extraction. This far, extracts obtained from peat with different extracting methods have been used as such without clear understanding of the compositions and without possibility to influence the proportions of the different fractions in the extract. It has not been possible to produce a peat extract with clearly defined composition, where the amount of important bioactive ingredients could be tailored according to the demands of the end use. Peat extracts have contained bioactive ingredients in same proportions, in which they exist originally in peat. No method of modification of these proportions has been proposed.

The object of the present invention is to solve or minimise the problems and disadvantages existing in the prior art.

One object of the present invention is to control the extract composition obtained by carbon dioxide peat extraction.

Another object of the present invention is an extract with clearly defined composition.

In order to achieve the above-mentioned objects the present invention is characterised in what is defined in the characterising parts of the independent claims presented hereafter.

In a typical method of extract composition control in extraction of peat by carbon dioxide the extraction temperature is 5 - 120 °C and pressure 70 - 1000 bar and the extraction is conducted in presence of a modifier.

A typical peat extract according to the present invention comprises 1 - 60 weight-%, preferably 1 - 20 weight-% of sterols.

Now it has been surprisingly found out that by using carefully chosen conditions during carbon dioxide extraction of peat composition of the obtained extract can be controlled and modified according to the specific needs of the end use. Important parameters with which the extract composition can be controlled and modified are the extraction pressure and temperature as well as the type and the concentration of modifier that is used in the extraction together with carbon dioxide.

The present invention can be applied to extraction of peat with carbon dioxide, either at sub- or supercritical conditions, preferably supercritical condition. Using carbon dioxide instead of organic solvents, the process has clear advantages of solvent free extract, safety, low cost and environmental friendliness. Supercritical carbon dioxide extraction is carried out in a system substantially free of oxygen or under low oxygen pressure. As the extraction conditions are generally mild the bioactive components and ingredients can be extracted in their native forms and the extract obtained free of harmful organic solvent residues.

The term "peat" when used in the context of the present invention relates to a material consisting largely of organic residues accumulated as a result of incomplete decomposition of dead plant constituents under conditions of excessive moisture with the aid of a wide spectrum of microbes. The abundance of microflora is a special feature of peat.

The term "skincare product" when used in the context of the present invention relates to moisturising and anti-wrinkle face creams, aftershave creams, moisturising and anti-wrinkle eye creams, moisturising and softening hand and foot creams, face and body creams for anti-inflammation caused e.g. by UV-irradiation, face and body creams for atopic diseases, face and body creams for preventing skin cancer induced e.g. by UV-irradiation and to sun creams for protecting skin from UV damage.

The term "keratin fibre care product" when used in the context of the present invention relates to shampoos, conditioners and balsam products for hair, hair styling products, such as mousses, waxes and gels, hair colours and toners, hair oils and hairsprays, shaving creams, beard and moustache colours and toners, as well as beard and moustache waxes. The term "keratin fibre" comprises both hairs of human and animal origin. The peat extract according to present invention can also be applied to products meant for animals, for example for pets, such as dogs or cats.

The term "modifier" when used in the context of the present invention relates to different solvents, such as different oils, water, acetone, different alcohols or mixtures of these. Examples of suitable oils are different vegetable oils like rapeseed or sunflower seed oils or the like and examples of suitable alcohols are methanol or ethanol. The term "modifier" is used in the context of the present application interchangeably with the term "co-solvent".

It is desirable to develop a control method for the extraction process of peat, so that important bioactive ingredients such as plant sterols, steryl esters, long-chain alcohols and long-chain fatty acid esters, can be selectively isolated from peat and/or enriched into obtained extract. These selectively isolated and/or enriched bioactive extracts can be used as ingredients for skin and keratin fibre care.

The present invention relates to a method of control of an extract composition, especially method of control of sterol content, in extracts obtained from non-processed and processed peat by means of carbon dioxide extraction with co-solvents and/or modifiers. The present invention relates especially to a method of control of an extract composition in extracts obtained from non-processed and processed peat by means of supercritical carbon dioxide extraction with co-solvents and/or modifiers. The extracts according to present invention comprise carefully and selectively isolated groups of lipophilic compounds, whose amounts can be controlled to desired levels.

Usually the obtained extracts according to one aspect of the invention are especially enriched with sterols, steryl esters or other sterol derivatives, long-chain alcohols and/or long-chain fatty acids. The long-chain alcohols and fatty acids have preferably main chain length of 16 to 40 carbon atoms. Typically the peat extract comprises 10 - 60 weight-%, preferably 20 - 40 weight-% of longs chain alcohols and 10 - 60 weight-%, preferably 10 - 40 weight-% long-chain fatty acids. According to one embodiment of the invention the peat extract can comprise up to 70 weight-% long chain alcohols. The major alcohols are usually octadecanol, eicosanol, docosanol, tetracosanol, hexacosanol, and octacosanol.

In addition, wax esters, acylglycerols and their derivatives as well as some special fatty acids such as hydroxyl fatty acids can be present in the extracts. The dominating fatty acids are usually palmitic, stearic, eicosanoic, docosanoic, tetracosanoic, pentacosanoic, hexacosanoic, and octacosanoic acids. Typically the peat extract comprises 30 - 50% wax esters comprising esters of alcohols and fatty acids with chain-length of typically 20 - 30 carbon atoms. The long-chain alcohols and long-chain fatty acids are normally in free form or in the form of wax esters.

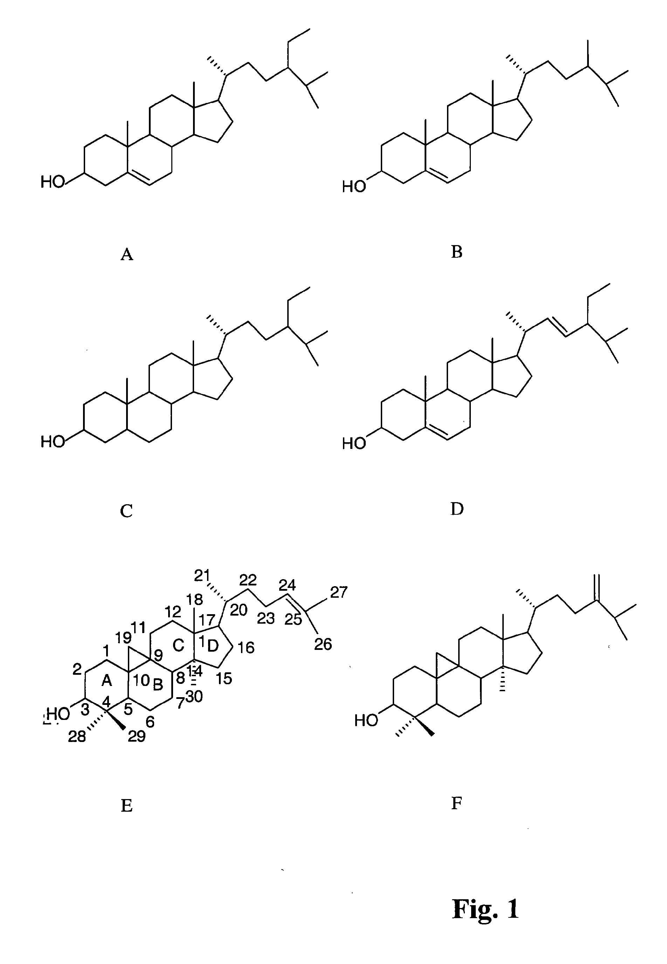

By using the present invention the total sterol content can be increased up to 60 weight-% of the extract. Typically the total sterol content can be increased up to 20 weight-% of the extract. According to one embodiment of the invention the sterol content in peat extract can be increased up to 10 weight-%. The major sterols found in the extract are sitosterol, campesterol, sitostanol, stigmasterol, cycloartenol and 24- methylenecycloartanol. The structures of sitosterol (A), campesterol (B), sitostanol (C), stigmasterol (D), cycloartenol (E) and 24- methylenecycloartanol (F) are shown in Figure 1. The sterols are normally in form of free sterols, steryl esters, or acylated steryl glycosides. In comparison it can be stated that the corresponding sterol content in extracts obtained with conventional uncontrolled processes is normally lower than 1%.

The nature of the extraction process and of the control method according to the present invention is non-destructive, which makes it possible to isolate the compounds in peat without causing any changes in their chemical structure during the extraction. Use of the method according to the present invention enables the production of peat extracts, which differ from extracts produced with conventional methods in both chemical composition and physical properties. The extracts obtained are usually light-coloured, free-flowing powders having a high content of sterols, long chain alcohols and/or fatty acids.

Peat or pre-treated peat, from which is used as raw material in the extraction process, can be of various compositions. Differences in the composition relate to the differences in peat forming plants and to differences in the processes, which have turned them into peat. The composition of fractions isolated can also be influenced by the choice of peat and/or pre-treated peat from which the fractions are isolated. According to one preferred embodiment of the invention the peat, which is used as a raw material in the extraction, is highly decomposed peat, such as grade 7 - 8 von Post humification scale, from e.g. *Spagnum* and *Carex* plants.

According to the present invention by adjusting of the extraction temperature, pressure as well as the types and concentrations of the modifiers used, the solvating property of carbon dioxide, preferably supercritical carbon dioxide, can be modulated considerably, especially in the region close to the critical temperature or pressure. This will yield extracts, wherein sterol content can be adjusted according to the needs. The selectivity of the method can be controlled by extraction pressure and temperature as well as by application of co-solvents and/or modifiers. These parameters govern the solvating power of carbon dioxide, preferably supercritical carbon dioxide, for different groups of compounds.

The extraction pressure varies according to the present invention from 70 to 1000 bar, preferably from 480 to 900 bars. The extraction temperature varies from 5 to 120 °C, preferably from 40 to 80 °C.

According to the present invention a modifier is added to influence the solubility of specific compounds during extraction. Most of the modifiers are used for increasing the polarity of CO₂ and thus solubility of certain slightly more polar components.

The concentration of modifiers should be adjusted according to the polarity of the modifier and target solutes to be extracted.

According the present invention the sterol content in the obtained extract can be adjusted to a level ranging from 1 to 60 weight-%, typically from 1 to 20 weight-%, by modifying the above-mentioned process parameters, i.e. temperature, pressure, type or concentration of a modifier. For example, when increasing extraction temperature from 20 °C to 60 °C and extraction pressure from 100 bars to 500 bars, the yield of total sterols and long chain alcohols are significantly increased. Use of water as modifier, in amounts ranging from 0.1 - 5 weight-%, typically 0.5 - 1 weight-%, will increase the yield of components with slightly higher polarity such as free sterols. According to one embodiment of the invention the amount of modifier varies between 1 - 3 weight-%.

Since peat is a complex of biomass, the extraction process according to present invention also reduces the content of microbes in the extract to a minimum level. A high content of microbes would limit the application of the extract, therefore the extraction procedure reduces the amount of microbes in the extracts to a minimum level, by adjusting the density of supercritical CO₂ and the particle size of the peat raw material. For example, under extraction conditions around 60 °C and 500 bars the microbes will be partially inactivated and remain mainly with the extraction residues.

The method according to invention can also include a pre-treatment of peat and/or post-extraction separation. According to one embodiment of the invention the peat is processed by drying, heating, milling, pelletising before the extraction. A preferred pre-treatment is pelletising, which often improves the economics of the process significantly due to the improved penetration and flow of carbon dioxide through the raw material.

The present invention can be carried out in a single autoclave or in two or several autoclaves connected in various configurations to each other e.g. in parallel and/or in series. Two, three or several autoclaves may, for example, be connected to each other to enhance the capacity and profitability of the process. Extraction can be carried out at any scale from laboratory scale and pilot-scale to various full-scale industrial processes; and it can be conducted as a batch-wise, semi-continuous or continuous process. It is possible to intensify the extraction process using e.g. supporting materials, such as diatomaceous earth, which can be mixed with raw materials to enhance the extraction efficiency. The extraction process according to the invention is also faster than common solvent extraction used previously due to e.g. a low number of unit processes. Process steps can also include feeding of peat or pre-treated peat using any feeding system, e.g. screws, belts or conveyors.

The obtained peat extract can be isolated from the extraction process by e.g. transferring the extract from the extraction system(s) with fluids or gases into separators or depressurising devices. A separator may be a dual separator in which the extract can be fractionated at different temperatures and pressures to achieve additional fractionation of the product. Specific fractions in the obtained extract can be isolated by purifying or fractionating the original extract, removing the more soluble compounds in the original extract with fluid or gas. This can be done in either a dynamic or static process in an autoclave or autoclaves or in fractionation or separation columns with e.g. a counter-current extraction process.

Extracts obtained from peat by the method according to the invention are preferably odourless solid powders, which are free of organic solvent and microbes and have a light, yellow-white colour. These powders are typically grainy and free-flowing when dry, which makes their handling and use easy. Thus they are especially useful as medicinal and/or cosmetic products or as ingredients in such products.

The extract according to the invention comprises plant sterols, plant steryl esters, long-chain alcohols, long-chain fatty acids and wax esters. These compounds are either direct constituents or molecular units of the lipids of skin barrier structure controlling the permeability of water, lipids as well as water- and lipid-soluble substances through skin. For example long-chain alcohol and long-chain fatty acids are molecular units of ceramides, an important component of skin barrier lipids. A preferable composition of the lipids in the skin barrier system is advantageous for the health and for the normal functions of skin, such as water-retaining capability and sensitivity of skin. When applied topically, the extract according to the present invention can provide skin with nutrients essential for building the barrier system and also have a series of beneficial physiological effects on skin.

Typically an extract according the present invention comprises 40 - 70 weight-% sitosterol, 10 - 20 weight-% campesterol, 2 - 5 weight-% cycloartenol and 2 - 5 weight-% 24-methylenecycloartanol, all calculated as proportion of the total sterols. The method according to the present invention can be made specific compared to e.g. organic extraction processes.

Peat extract according to the present invention can be used in skin care or keratin fibre care products, general hygiene products and/or cosmetic products. The amount of the peat extract in the different products is 0.1 - 99.9 weight-%, typically 1 - 90 weight-%, more typically 5 - 40 weight-%.

Thus, according to one preferred embodiment of the present invention the sterol-enriched peat extract, is used as bioactive ingredient in cosmetics, skincare products for improving skin moisture, decreasing skin sensitivity, retarding skin aging, alleviating skin inflammation, reducing the risk of skin cancers, and improving the general appearance of skin surface. The peat extract can also be used as an ingredient or component in keratin fibre care products to improve the health and outlook of keratin fibres, such as human or animal hair.

Based on the skin-beneficial effects plant sterols and long-chain alcohols described in this application, the extracts according to present invention can furthermore be applied as bioactive ingredients in skincare and keratin fibre care products with functional targets of skin-moisturising, anti-inflammation, anti-skin cancer, decreasing skin sensitivity, regulating immune function, promoting tissue regeneration as well as improving the general health condition of skin.

According to one embodiment of the invention skin care product comprises peat extract obtained by a method according to the present invention at levels ranging from 0.1 - 99 weight-%, preferably 5 - 30 weight-%. The extract can be used as single bioactive ingredient or in combination with other ingredients such as natural and/or synthetic antioxidants and vitamins such as vitamin C, tocopherols and tocotrienols. Also other natural extracts or synthetic ingredients containing skin-beneficial compounds can be added. Preferably the extract according to the present invention is used in combination with other bioactive ingredients such as vitamins, antioxidants, proteins, and/or amino acids.

According to one embodiment of the invention the peat extract can be used in general hygiene products, such as antiperspirants, talcum powders, baby powders, facemasks, skin exfoliating products or washing agents, such as soaps and shower gels. According to one embodiment of the invention the amount of extract in the final product can be 0.1 - 99 weight-%, preferably 5 - 20 weight-%. The extracts can be used either as sole bioactive compounds or preferably in combination with other ingredients beneficial to skin.

According to another embodiment of the invention the peat extract can be used in other cosmetic products, such as lipsticks, lip-glosses, lip creams, mascaras, eyebrow pencils, kohl pencils, lip liners, cover sticks, blushers, foundation creams, face powders, eye shadows, cuticle creams, nail oils or colours. According to one embodiment of invention the amount of peat extract is usually 0.1 - 99.9 weight-%, preferably 20 - 40 weight-%, either as sole bioactive ingredient or preferably in combination with other beneficial ingredients such as fatty acids, vitamins C and E, and ceramides.

### Example 1

The invention was carried out as follows. Peat was dried and milled in a hammer mill. A volume of 100 1 of milled peat was placed in an extraction basket of 300 1, transferred in an autoclave and extracted by supercritical carbon dioxide at a pressure on 480 bar and at a temperature of 70 °C. The flow rate of the circulating fluid was 30 l/min. Extract was separated during the fluid circulation at lowered pressure in a separator at a pressure of 50 bars and temperature of 30 °C. The carbon dioxide was be re-circulated by pumps and heat exchangers back to the autoclave. Water was used as a modifier.

### Example 2

Highly decomposed peat of grade 7 - 8 von Post humification scale was extracted according to method of Example 1. The obtained product, i.e. extract contained up to 10 - 20 weight-% sterols and up to 60 - 70 weight-% long-chain alcohols and fatty acids. The extract had light yellowish colour and was devoid or nearly devoid of any smell and free of microbes.

### Example 3

Peat material was obtained from natural bogs and mires of selected locations in Finland. After being air-dried, the peat was milled to a particle size 0.2 - 0.9 mm and pelletised before extraction. The supercritical extraction was carried out under conditions of temperature 40 - 60 °C and pressure 300 - 500 bars. The extraction time was 1 - 2 hours. Water was used as modifier. The extract was dried at 40 °C to water content less than 10%.

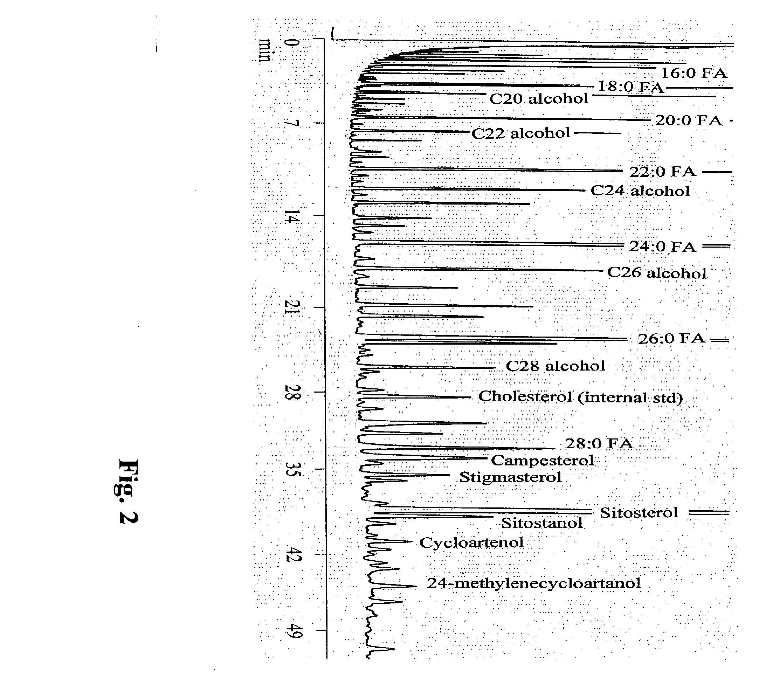

After saponification of the peat extract, the unsaponifiables were separated, and the long-chain alcohols, acids and sterols were analysed with GC-FID and GC-MS. A capillary column DB-1701 (30 m x 0.25 mm, d_{f} 0.20 µm) was used for GC analysis. In Figure 2 a typical GC-FID chromatogram of peat extract is shown. The column temperature program was 230 °C hold 1 min, followed by an increase to 275 °C at a rate of 1 °C/min, and hold at 275 °C for 30 min. The injector temperature was 280 °C, and detector temperature 300 °C.

### Example 4

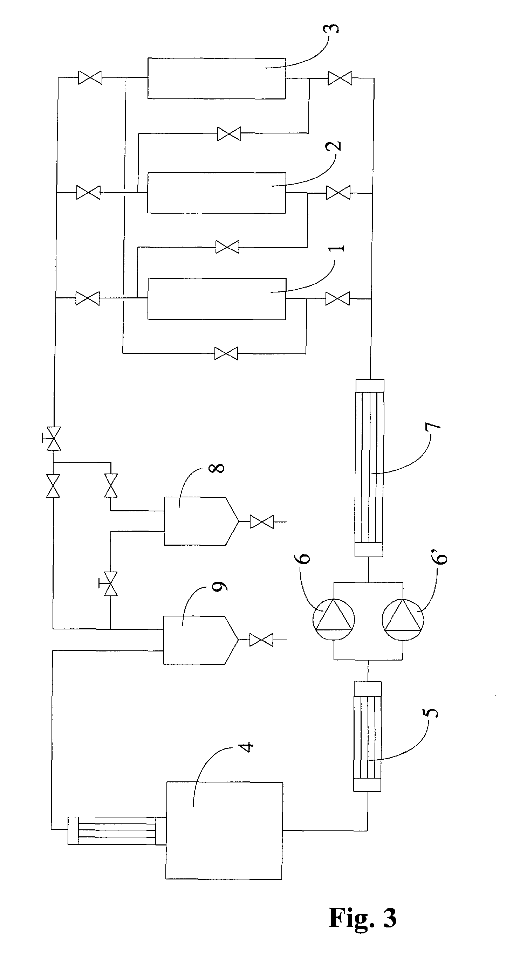

One embodiment of the present invention is shown in Figure 3, which shows a flow diagram for supercritical fluid extraction utilising carbon dioxide and using milled peat as raw material. In Figure 1 the milled peat is fed batch wise into autoclaves, i.e. extractors 1, 2, 3. Carbon dioxide is fed from the carbon dioxide storage tank 4 through a sub-cooler 5 and pumped with pumps 6, 6' through a pre-heater 7 to the extractors 1, 2, 3, which are set up in series. Extract is fractioned in two successive decompression stages and the extracted fractions are collected from separators 8, 9. The carbon dioxide can be returned to the storage tank 4.

It will be appreciated that the present invention can be incorporated in the form of a variety of embodiments, only a few of which are disclosed herein. It will be apparent for the specialist in the field that other embodiments exist and do not depart from the spirit of the invention. Thus, the described embodiments are illustrative and should not be construed as restrictive.

### REFERENCES

Kashibuchi, N; Matsubara, K; Kitada, Y; Suzuki, H; (1993) New treatment of dry skin by controlling stratum corneum turnover rate. Journal of SCCJ, 27 (3), 383-393.

Mironov, VA; Guseva-Donskaya, TN; Dubrovina, YuYu; Osipov, GA; Shabanova, EA; Nikulin, AA; Amirov, NSh; Trubitsina, IG; (1989) Khimiko-Farmatsevticheskii Zhurnal, 23 (11), 1357-1364.

Mora, M.P.; Tourne-Peteilh, C.; Charveron, M.; Fabre, B.; Milon, A.; Muller, I. (1999) Optimization of plant sterols incorporation in human keratinocyte plasma membrane and modulation of membrane fluidity. Chemistry and Physics of Lipids, 101 (2), 255-265.

## Claims

1. Method of extract composition control in peat extraction by supercritical carbon dioxide, in which method the extraction temperature is 5 - 120 °C and pressure 70 - 1000 bar **characterised in that** the extraction is conducted in presence of a modifier.

2. Method according to claim 1, **characterised in that** the modifier is selected from the group comprising different oils, water, acetone and/or different alcohols.

3. Method according to claim 1, **characterised in that** the extraction is conducted in pressure interval ranging from 480 to 900 bar.

4. Method according to claim 1, **characterised in that** the extraction is conducted in the temperature interval ranging from 40 to 80 °C.

5. Peat extract **characterised in that** the extract comprises 1 - 60 wt-%, preferably 1 - 20 wt-% sterols.

6. Peat extract according to claim 5, **characterised in that** the extract comprises 10 - 60 wt-%, preferably 20 - 40 wt-% long-chain alcohols, and 10 - 60 wt-%, preferably 10 - 40 wt-% long-chain fatty acids.

7. Peat extract according to claim 5, **characterised in that** the extract comprises 40 - 70 wt-% sitosterol, 10 - 20 wt-% campesterol, 2 - 5 wt-% cycloartenol and 2 - 5 wt-% 24-methylenecycloartanol, calculated from total sterol content.

8. Peat extract according to claims 6 or 7, **characterised in that** it is obtained by using a method described in any of the claims 1 to 4.

9. Use of peat extract according to any of the claims 6 - 8 in skin care or keratin fibre care products, general hygiene products and/or cosmetic products.

10. Use according to claim 9, **characterised in that** the amount of the peat extract in the product is 0.1 - 99.9 weight-%, typically 5 - 40 weight-%.
